# EUROPEAN PATENT APPLICATION

(11) **EP 2 664 307 A1**
(43) Date of publication of application: **20.11.2013**
(21) Application number: 13167548.0
(22) Date of filing: 13.05.2013
(51) Int. Cl.: A61F 5/02, A44B 11/12

(54) **Easily adjustable hyperextension orthopaedic corset**

(30) Priority: 14.05.2012 IT MI20120830
(71) Applicant: Orthoservice AG, 6362 Stansstad (NW) (CH)
(72) Inventor: Rossi, Paolo, 6362 Stansstad (NW) (CH)
(74) Representative: Branca, Emanuela

(57) **Abstract**

The present invention relates to an easily adjustable hyperextension orthopaedic corset comprising a frame structure consisting of a sternal rod (12) extending substantially horizontally, a pair of side uprights (18) extending substantially vertically and a lower pelvic strap (14), substantially horizontal, that connects the pair of side uprights (18), a plurality of contact plates (26,28) being adjustably constrained to the frame structure to form contact surfaces with a patient's torso, the corset (10) also comprising a tensioning system comprising a rear belt (30) onto which a lumbar plate (32) can be inserted, the rear belt (30) being constrainable at its opposite ends (30a,30b) to the pair of side uprights (18) respectively by means of a releasable attachment means (34) to which the rear belt (30) is connected at one first end thereof (30a) so as to be adjustable in length, and a tensioning lever (40) to which the rear belt (30) is connected at one second end (30b), and is characterized in that the rear belt (30) is connected to the tensioning lever (40) so as to be adjustable in length.

## Description

The present invention relates to an easily adjustable hyperextension orthopaedic corset, which can be used for the treatment of moderate and serious dorsolumbar pathologies.

A number of pathologies or sequelae of trauma require stabilization of the spinal column and an anti-gravitational action on the spine, which allows reduction of the load of the upper part of the torso, which, due to gravity, normally unloads precisely along the spine.

The pathologies in question are sequelae of trauma, such as vertebral fractures, tumour pathologies, which can lead to manifestations of vertebral metastases with consequent increased porosity of the affected bone structure, or osteoporosis due to ageing, which can lead to vertebral collapses associated with compression of the nerve endings.

Immobilization and reduction of the load on the spine are currently achieved through the use of a so-called hyperextension corset, which the patient must always wear when in the upright position. Indeed, in this circumstance, the load on the upper part of the torso is entirely borne by the spinal column.

The hyperextension orthopaedic corset consists of a frame structure made of light aluminium alloy that, when worn over the torso, offers two thrust points in the front part, identifiable in the chest area (sternum) and on the pelvis, and a thrust point in the rear part (indicatively at the dorsolumbar passage), created with a belt constrained to the aluminium structure.

To improve patient comfort, the resting points on the torso and on lumbar fascia consist of padded plates, which guarantee the advantageous distribution of the pressure over a more extensive body surface. Hyperextension orthopaedic corsets are normally available in various sizes, but must nevertheless be perfectly adjusted and adapted on the basis of the morphology of each individual patient.

In order to wear the hyperextension corset, as well performing the adjustments to the frame structure, the lumbar plate must be correctly positioned and fastened by means of the rear belt so as to be tensioned.

To this end, in the known hyperextension orthopaedic corsets, a first end of the rear belt is fixedly constrained to a first upright of the frame structure through a tensioning lever, and the second end is adjustably connected, so as to be adjustable in length, to attachment means to the opposite upright of the frame structure. The length of the rear belt is adjusted by acting at the point of attachment of the belt and the upright.

To be adaptable to a wide range of physiques in the context of a same size, the above-mentioned adjustment should possibly permit a wide variability in the length of the belt, quantifiable in terms exemplary in 25 cm approximately.

The currently known hyperextension orthopaedic corsets do not allow to achieve a very broad variability without, on the other hand, introducing drawbacks. Indeed, for particularly narrow adjustments, the excess portion of belt or of means for adjusting would form an abundance of material that would remain free and accumulated at the second end of the belt, thus causing discomfort to the user and making the orthopaedic corset particularly unpleasant to wear.

In addition, the user would also find considerable inconvenience from the aesthetic point of view, encountering difficulties in concealing the abundance of material from view, particularly if wearing light clothing.

Not least, in the event of adjustments of the rigid type, for example achieved through a portion of rigid toothed belt that engages with the upright attachment means, it would not be possible to guarantee, for all the wide range of adjustments, a centred positioning of the lumbar plate, which indeed cannot slide at the portion of toothed belt.

The aim of the present invention is therefore to produce an improved hyperextension orthopaedic corset which allows a wide variability of adjustment of the length of the rear belt.

Another aim of the invention is to produce an improved hyperextension orthopaedic corset wherein for each permitted adjustment of the rear belt there is no inconvenience to the user wearing the corset in terms of comfort and aesthetics.

A further aim of the present invention is to produce an improved hyperextension orthopaedic corset wherein, for each permitted adjustment of the rear belt, it is possible to maintain the lumbar plate in a centred position.

Yet another aim of the present invention is to produce an improved hyperextension orthopaedic corset that is particularly simple and functional and cost-effective. These aims according to the present invention are achieved by producing an improved hyperextension orthopaedic corset as set forth in claim 1.

Further characteristic of the invention are highlighted in the dependent claims, which are an integral part of this description.

The characteristics and the advantages of the improved hyperextension orthopaedic corset according to the present invention will become clearer from the following description, given by way of a non-limiting example, in relation to the accompanying schematic drawings, in which:

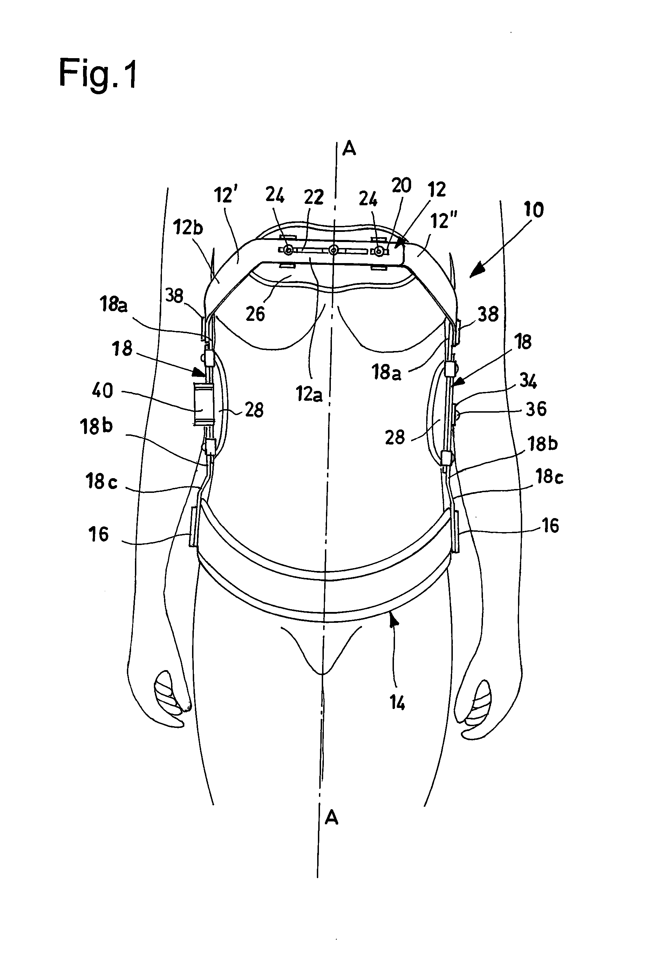

- figure 1 is a front view of an hyperextension orthopaedic corset when worn;

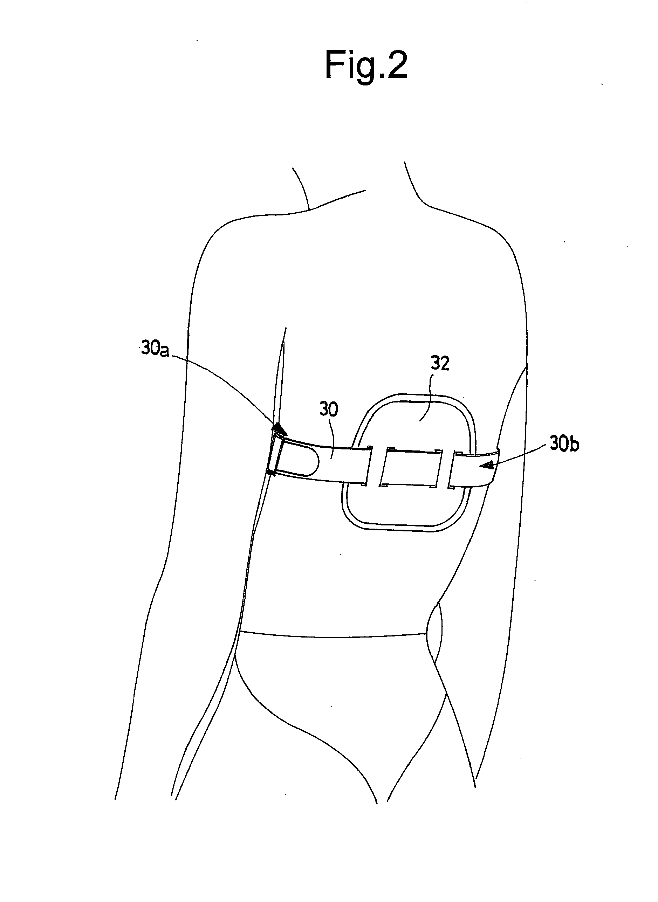

- figure 2 is a rear view of the hyperextension orthopaedic corset of figure 1;

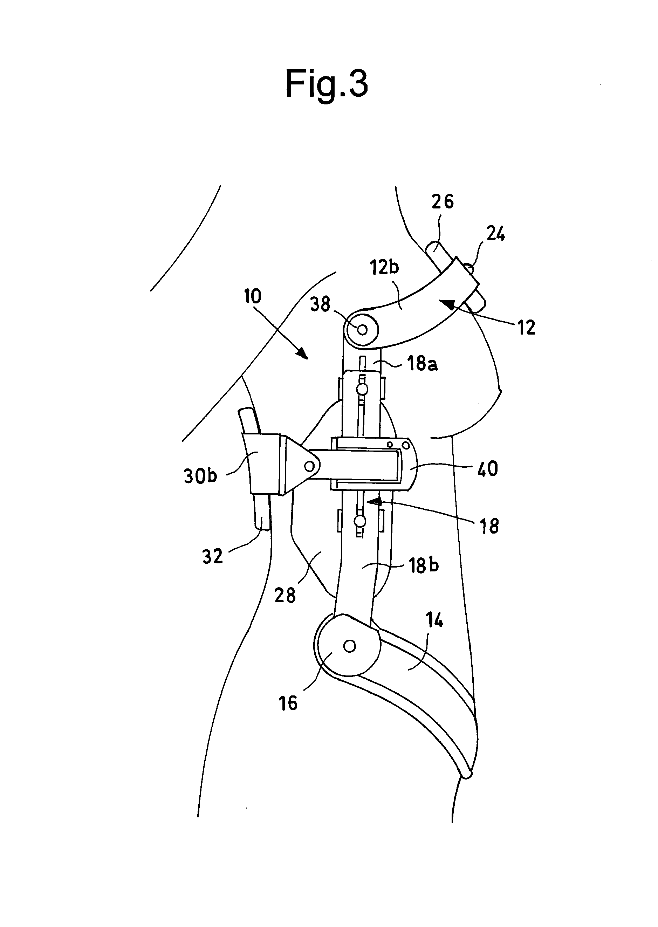

- figure 3 is a side view of the hyperextension orthopaedic corset of figure 1;
- figure 4 is a perspective view of the tensioning lever of the hyperextension orthopaedic corset of figure 1;
- figures 5a, 5b and 5c are perspective views of the tensioning lever of figure 4 in open configuration, respectively in an exploded view, with the belt adjusted long and with the belt adjusted short;
- figures 6a and 6b are cross-sectional views of the tensioning lever of figure 4 in closed configuration, respectively with belt adjusted long and with belt adjusted short.

With reference to the figures, an improved hyperextension orthopaedic corset according to the present invention is shown, globally indicated by the reference number 10.

The corset 10 consists of a frame structure made of light aluminium alloy that is equipped with relative movements for adjusting chest width, torso height and positioning on the pelvis.

Padded plates 26,28,32 which form the contact surfaces with the patient's torso, and a tensioning system for tensioning the corset 10 when worn, are applied onto the frame structure by means of suitable connection elements, as will be better specified below.

The frame structure consists of rods having substantially horizontal development, located at the sternum and at the pelvis and respectively called sternal rod 12 and pelvic rod 14. The sternal rod 12 and pelvic rod 14 are spaced apart by substantially vertical side uprights 18.

In particular, a sternum contact plate 26 is applied to the sternal rod 12, said plate being preferably made of aluminium, padded and suitably shaped with anatomical form, which is permitted an oscillatory adaptation motion to the inclination of the sternum wall. Respective padded side plates 28 are applied to the side uprights 18, said plates also having an anatomical form so as to better adapt to the hips and protect them from contact with the side uprights 18.

The sternal rod 12 consists of a pair of semi-rods 12',12", which each have a straight section 12a and a curved section 12b, so as to follow the shape of the chest.

Each straight section 12a of the sternal semi-rods 12',12" is equipped with a rectangular window 20 in proximity of the end and with a groove 22, affecting the remaining straight portion.

The two straight sections 12a are mounted superimposed in a symmetrical position with respect to a sagittal plane A, so as to allow a relative translation movement.

The two sternal semi-rods 12',12" are kept coupled by constraining elements, consisting of two adjusting clips 24, which each contemporaneously engage with the rectangular windows 20 and with the grooves 22 of the semi-rods 12', 12".

The adjusting clips 24 are of a type known in the prior art and will not thus be described in further detail. Two upper side semi-uprights 18a, left and right, are applied to the sternal semi-rods 12',12", at the relative curved sides 12b, through coupling means 38 having adjustable inclination, preferably but not necessarily, stepped according to a discrete number of positions. For example, the connection between the sternal semi-rods 12',12" and the upper side semi-uprights 18a takes place by means of threaded fastening means that also allow the aforementioned angular adjustment.

Each upper side semi-upright 18a is also coupled, in such a way as to allow the adjustment in height, with a respective lower side semi-upright 18b so as to form the entire side upright 18 of the corset 10.

The lower side semi-uprights 18b end with a curved lower portion 18c, which follows the widening of the user's pelvis and in turn ends with an articulable connection element 16 to the pelvic strap 14, which constitutes the lower horizontal connection rod between the side uprights 18.

This pelvic strap 14 consists of a metal core covered with a padding and arched according to an anatomical form to adapt to the pelvis of the user of the corset 10.

The tensioning system comprises a rear belt 30 onto which is inserted a lumbar plate 32. This lumbar plate 32, suitably reinforced and padded, is the rear thrust point of the corset 10.

The rear belt 30 is constrainable, at its opposite ends 30a,30b, to the side uprights 18. To this end, a first end 30a of the opposite ends 30a,30b of the rear belt 30 is connected to an attachment means 34 at one of the side uprights 18, while a second end 30b of the opposite ends 30a,30b of this rear belt 30 is connected to a tensioning lever 40 that is fixedly constrained to the opposite side upright 18.

The attachment means 34 is provided with a hole (not illustrated) with form such as to allow the insertion therein and the locking in position of a protruding pin element 36 that is integral with one of the side uprights 18, thus forming a detachable connection of the rear belt 30 to the frame structure of the corset 10.

The rear belt 30 is connected to the attachment means 34 so as to be adjustable in length.

According to a preferred embodiment of the present invention, for the connection of the rear belt 30 to the attachment means 34 there are preferably provided toothed means (not illustrated) which allow a step adjustment in length, consisting of a toothed belt selectively engageable with one or more teeth obtained on the attachment means 34.

In alternative embodiments, the attachment means 34 is provided with loops (not illustrated) for the insertion and folding in of the rear belt 30 to form a ring in such a way as to adjust the rear belt 30 in length and prevent the sliding thereof 30 into the slots. According to the present invention, the rear belt 30 is also connected, so as to be adjustable in length, to the tensioning lever 40.

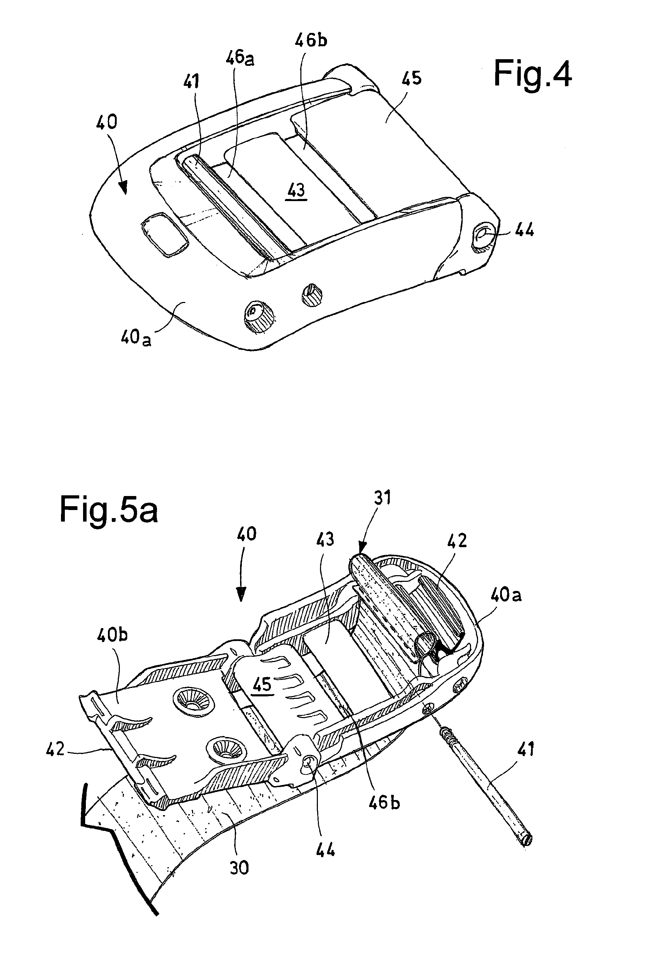

According to the preferred embodiment illustrated, this additional adjustment is obtained by means of the particular tensioning lever 40 illustrated in figures 4-6, which allows a rough preliminary adjustment of the length of the rear belt 30 to be performed.

This tensioning lever 40 comprises an upper portion 40a substantially produced with U-shaped body and a lower portion 40b that is substantially plate-like, respectively pivoted on a first side through pivotable constraining means 44, so as to produce a shell closing/opening of the lever 40.

The upper portion 40a and the lower portion 40b of the tensioning lever 40 further comprise, on the side opposite to the first side, removable coupling means 42 adapted to produce a releasable constraint between these upper 40a and lower 40b portions, in order to keep the two portions 40a, 40b contiguous when the lever 40 is in the closed configuration, contrasting any forces due to the tensioning of the rear belt 30. The lower portion 40b of the tensioning lever 40 is integrally constrained to one of the side uprights 18. The upper portion 40a of this tensioning lever 40 is connected to the rear belt 30 through a removable pin 41 which engages in a ring-shaped portion 31 produced in the second end 30b of the rear belt 30. The ring portion 31 may be produced by stitching or in another suitable manner.

The removable pin 41 is transversely arranged with respect to the two limbs of the U-shaped body of the upper portion 40a of the tensioning lever 40 and substantially in proximity to the coupling means 42. The tensioning lever 40 also comprises, at the pivotable constraining means 44, a first end rod 45, which is also transversely arranged with respect to the two limbs of the U-shaped body.

A second intermediate rod 43 is further provided, also transversely arranged with respect to the two limbs of the U-shaped body, interposed to the removable pin 41 and to the end cross-rod 45 and spaced apart therefrom in order to produce a pair of passages 46a,46b for the rear belt 30.

Passages 46a,46b preferably have dimensions such as to allow the passage with interference of the rear belt 30 in order to contrast the sliding thereof.

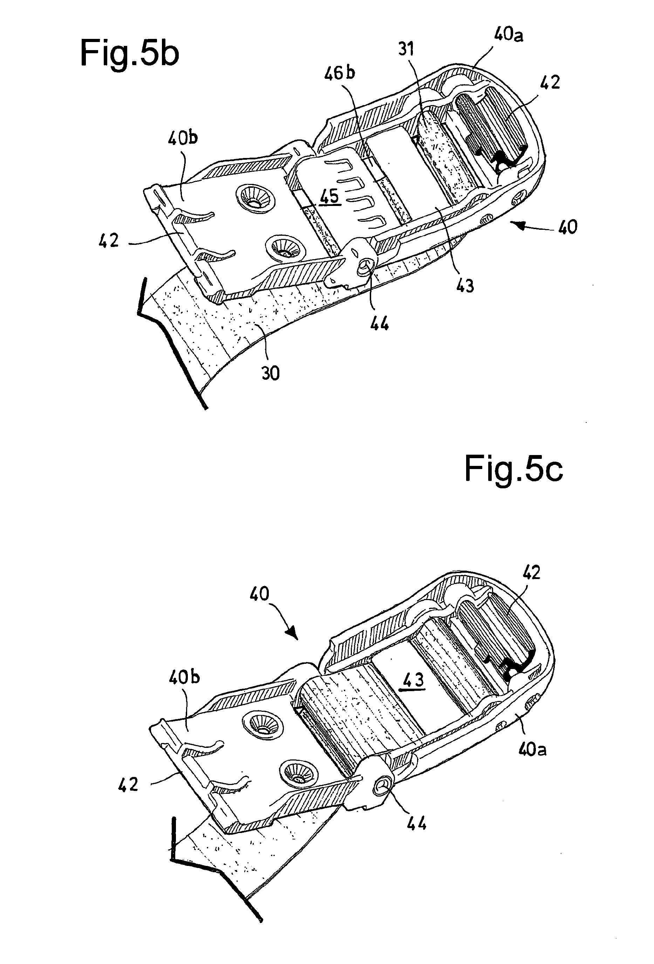

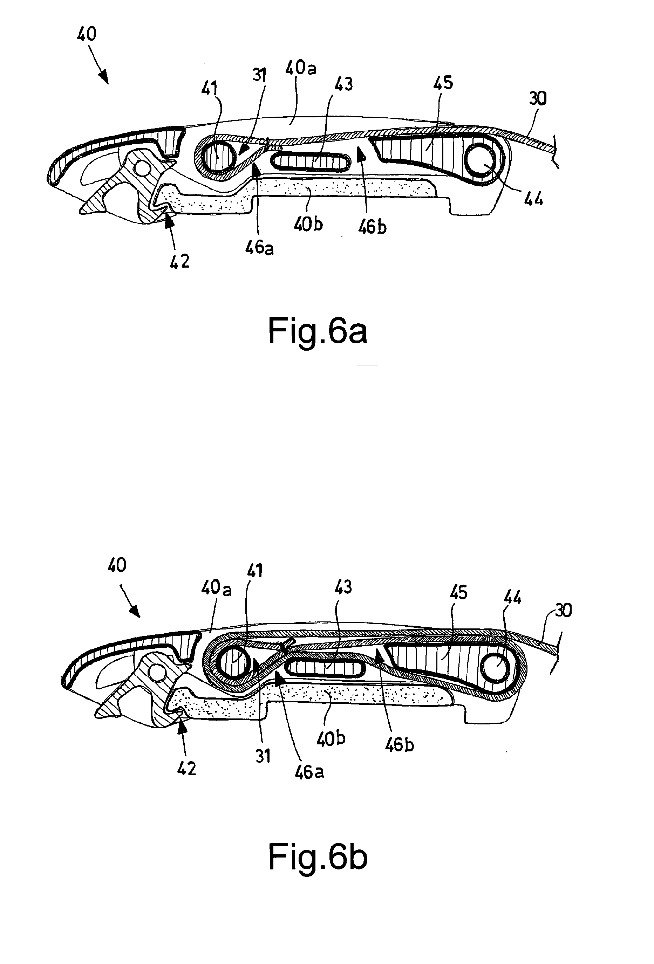

It is therefore possible to constrain the rear belt 30 to the tensioning lever 40 according to two configurations that differ from each other due to the resulting length of the belt 30: a first long configuration, shown in figures 5b and 6a, and a second short configuration, shown in figures 5c and 6b.

In the first long configuration, the rear belt 30 is exclusively constrained to the removable pin 41 through the end ring 31.

In the second short configuration, the rear belt 30 is wound around the cross-rods 43,45 and the removable pin 41. In particular, the rear belt 30 constrained to the lever 40 through the removable pin 41 at its end ring 31, is passed around the end cross-rod 45, above the intermediate rod 43 and back to the removable pin 41, around which it passes.

This path of the belt 30, in addition to leading to an overall shortening, prevents relative sliding thanks to the passage with interference through the passages 46a,46b interposed between the cross-rods 43,45 and at the removable pin 41.

It is clear that, in case it is necessary to operate a particularly narrow adjustment, the rear belt 30 can be connected to the tensioning lever 40 in short configuration (figures 5c and 6b) so that a wide adjustment in length is not necessary at the attachment means 34.

On the contrary, for particularly wide adjustments, the rear belt 30 will be connected to the tensioning lever 40 in long configuration (figures 5b and 6a).

The application operations of the hyperextension corset 10 thus consist of a preliminary, rough adjustment of the length of the rear belt 30 which provides for the setting of the connection of the belt 30 to the closure lever in long or short configuration, and of a successive fine adjustment of the length of the rear belt 30, performed, in a known manner, at the attachment means 34.

The attachment means 34 of the rear belt 30 is subsequently coupled to the protruding pin element 36, so that this rear belt 30 adheres to the body, but is not tensioned, and that the closure lever 40 is in the open position. Lastly, by activating the closure lever 40 it is possible to tighten the rear belt 30 and obtain the correct application of the hyperextension corset 10.

It has thus been seen that the improved hyperextension orthopaedic corset according to the present invention achieves the previously-stated aims.

The improved hyperextension orthopaedic corset of the present invention thus devised is, in any case, susceptible to a number of modifications and variants, all of which fall under the same inventive concept.

As an example, the upper portion of the tensioning lever can be equipped with a single cross-rod - the end one for example - thereby achieving a single passage for the folding in of the rear belt.

Moreover, all the details can be replaced by technically equivalent elements. In practice, any materials as well as any shapes and dimensions can be used depending on the technical requirements.

The scope of protection of the invention is therefore defined by the accompanying claims.

## Claims

1. An hyperextension orthopaedic corset (10) comprising a frame structure consisting of a sternal rod (12) extending substantially horizontally, a pair of side uprights (18) extending substantially vertically and a lower pelvic strap (14), substantially horizontal, that connects said pair of side uprights (18), a plurality of contact plates (26,28) being adjustably constrained to said frame structure to form contact surfaces with a patient's torso, the corset (10) also comprising a tensioning system comprising a rear belt (30) onto which a lumbar plate (32) can be inserted, said rear belt (30) being constrainable at its opposite ends (30a,30b) to said pair of side uprights (18) respectively by means of a releasable attachment means (34) to which said rear belt (30) is connected at one first end thereof (30a) so as to be adjustable in length, and a tensioning lever (40) to which said rear belt (30) is connected at one second end thereof (30b), **characterized in that** said rear belt (30) is connected to said tensioning lever (40) so as to be adjustable in length.

2. The hyperextension orthopaedic corset (10) according to claim 1, **characterized in that** said tensioning lever (40) comprises a substantially U-shaped upper portion (40a) and a substantially plate-shaped lower portion (40b) integrally constrained to one of said side uprights (18), said upper portion (40a) and said lower portion (40b) being respectively hinged at a first side through pivotable constraining means (44), said upper portion (40a) comprising a removable pin (41) arranged transversally with respect to the two limbs of the U-shaped body for engagement in a ring portion (31) located at said second end (30b) of said rear belt (30), and an end rod (45) also arranged transversally with respect to the two limbs of the U-shaped body and spaced apart from said removable pin (41) so as to form a first passage (46a) for said rear belt (30).

3. The hyperextension orthopaedic corset (10) according to claim 2, **characterized in that** said upper portion (40a) of said tensioning lever (40) comprises a second intermediate rod (43) also arranged transversally with respect to said two limbs of said U-shaped body of said upper portion (40a), said second intermediate rod (43) being interposed between said removable pin (41) and said end cross-rod (45) and spaced apart from the same (41,45) so as to form a pair of passages (46a,46b) for said rear belt (30).

4. The hyperextension orthopaedic corset (10) according to claim 2 or 3, **characterized in that** said upper portion (40a) and said lower portion (40b) of said tensioning lever (40) comprise, at an opposite side with respect to said first side, removable coupling means (42) suitable for forming a releasable constraint between said upper portion (40a) and said lower portion (40b) in order to keep said two portions (40a, 40b) contiguous when said tensioning lever (40) is in a closed configuration.

5. The hyperextension orthopaedic corset (10) according to claim 4, **characterized in that** said removable pin (41) is substantially located close to said coupling means (42) and said end rod (45) is substantially located at said pivotable constraining means (44).

6. The hyperextension orthopaedic corset (10) according to any of the preceding claims, **characterized in that** said attachment means (34) is provided with a hole shaped so as to allow the insertion and locking in position of a protruding pin element (36) integral with one of said side uprights (18).

7. The hyperextension orthopaedic corset (10) according to any of the preceding claims, **characterized in that** said rear belt (30) is connected to said attachment means (34) by means of toothed means consisting of a toothed belt which can be selectively engaged with one or more teeth located on said attachment means (34).

8. The hyperextension orthopaedic corset (10) according to any of the preceding claims from 1 to 6, **characterized in that** said rear belt (30) is connected to said attachment means (34) by the insertion and folding in at least one loop formed in said attachment means (34).

9. The hyperextension orthopaedic corset (10) according to any of the preceding claims, **characterized in that** said sternal rod (12) and said pair of side uprights (18) are adjustable in length, respectively consisting of a pair of sternal semi-rods (12a,12b) and of a pair of side semi-uprights (18a,18b) having a relative adjustable translation movement.

10. The hyperextension orthopaedic corset (10) according to claim 9, **characterized in that** the lower side semi-uprights (18b) of said pair of side semi-uprights (18a,18b) end with a lower curved portion (18c) which in turn ends with an articulable connection element (16) to said pelvic strap (14).

11. The hyperextension orthopaedic corset (10) according to any of the preceding claims, **characterized in that** said sternal rod (12) and said pair of side uprights (18) are provided with mutual connection means (38) having adjustable inclination.
